(19) Europäisches Patentamt
European Patent Office
Office européen des brevets

(11) Veröffentlichungsnummer : **0 022 484**
**B1**

(12) # EUROPÄISCHE PATENTSCHRIFT

(45) Veröffentlichungstag der Patentschrift : **22.09.82**

(21) Anmeldenummer : **80103484.4**

(22) Anmeldetag : **21.06.80**

(51) Int. Cl.³ : **C 07 C 51/60**, C 07 C 63/15, C 07 C 63/22, C 07 C 63/24, C 07 C 63/30, C 07 C 63/38// B01J31/02

(54) **Verfahren zur Herstellung aromatischer Dicarbonsäuredichloride und ihre Verwendung bei der Herstellung von Polykondensaten.**

(30) Priorität : **03.07.79 DE 2926789**

(43) Veröffentlichungstag der Anmeldung : **21.01.81 (Patentblatt 81/03)**

(45) Bekanntmachung des Hinweises auf die Patenterteilung : **22.09.82 Patentblatt 82/38**

(84) Benannte Vertragsstaaten : **BE DE FR GB IT NL**

(56) Entgegenhaltungen :
**DE A 2 657 734**

**CHEMICAL ABSTRACTS, Band 84, Nr. 21, 24. Mai 1976, Zusammenfassung Nr. 150214w, Seite 510, Columbus, Ohio, US**

(73) Patentinhaber : **BAYER AG**
**Zentralbereich Patente, Marken und Lizenzen**
**D-5090 Leverkusen 1, Bayerwerk (DE)**

(72) Erfinder : **Freitag, Dieter, Dr.**
**Hasenheide 10**
**D-4150 Krefeld (DE)**
Erfinder : **Schmidt, Manfred, Dr.**
**1020 North-Third-Street**
**New Martinsville 26155 (US)**

**EP 0 022 484 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren zur Herstellung aromatischer Dicarbonsäuredichloride und ihre Verwendung bei der Herstellung von Polykondensaten

Die Erfindung betrifft ein Einstufen-Verfahren zur Herstellung von hochreinen polykondensationsfähigen aromatischen Dicarbonsäuredichloriden.

Die Erzeugung von aliphatischen und aromatischen Säurechloriden durch Reaktion einer Carbonsäure mit Phosgen ist in den US-PS 3 184 506, 3 544 626, 3 544 627, 3 547 960 sowie in den DE-OS 2 400 007 und 2 321 122 beschrieben. Nach diesen Verfahren werden als Reaktionsprodukte dunkelgefärbte Carbonsäurechloride in einer Reinheit von 96 bis 99 % erhalten. Aromatische Dicarbonsäuredichloride dieses geringen Reinheitsgrades können nicht direkt nach dem Zweiphasengrenzflächenpolykondensationsverfahren zur Herstellung von hochmolekularen Polykondensaten, wie aromatischen Polyamiden oder aromatischen Polyestern, eingesetzt werden. Ihr Gehalt an nicht oder nur halbseitig umgesetzten Dicarbonsäuren stört die Polykondensation, verursacht unerwünschten Kettenabbruch, und ergibt Polymerisate mit endständigen Carboxylgruppen. Die so hergestellten aromatischen Dicarbonsäuredichloride sind durch Verunreinigungen dunkel gefärbt und enthalten durch Umsetzung mit den Katalysatoren entstandene störende Carbamidsäurechloride (vergl. Chem. Ref. 1973, Vol. 73, Nr. 1, Seite 77 oder Angewandte Chemie (1974), Jahrg. 1962, Nr. 21, Seite 864).

Um farblose Dicarbonsäuredichloride zu erhalten, müssen die Rohprodukte durch Umkristallisation oder Destillation gereinigt werden. Dies erfordert zusätzlichen Aufwand und vermindert die Ausbeute ; bei aromatischen Dicarbonsäuredichloriden besteht die Gefahr einer spontanen Zersetzung.

Gegenstand der Erfindung ist ein Einstufen-Verfahren zur Herstellung von reinen aromatischen Dicarbonsäuredichloriden durch Umsetzung einer aromatischen Dicarbonsäure mit Phosgen in Anwesenheit eines Katalysators und gegebenenfalls in einem Lösungsmittel und/oder Verdünnungsmittel, das dadurch gekennzeichnet ist, daß man als Katalysatoren Phosphinimine oder Phosphorimidate oder deren Mischungen verwendet. Die so erhaltenen aromatischen Dicarbonsäuredichloride sind praktisch farblos und enthalten neben den eingesetzten Katalysatoren 0,1 % Verunreinigungen oder weniger, so daß sie ohne Nachreinigung zur Herstellung von farblosen, hochmolekularen Polykondensaten eingesetzt werden können.

Die erfindungsgemäß als Katalysatoren eingesetzten Phosphinimine oder Phosphorimidate oder Gemische von Phosphiniminen oder Phosphorimidaten stören beispielsweise die Herstellung von aromatischen Polyestern nicht. Bei den Waschvorgängen im Anschluß an das Polykondensationsverfahren nach der 2-Phasengrenzflächenmethode werden die Katalysatoren durch die wäßrig-alkalische Phase von der organischen Polymerlösung abgetrennt.

Als erfindungsgemäße Katalysatoren sind Phosphinimine der allgemeinen Struktur (I) und Phosphorimidate der allgemeinen Struktur (II) geeignet.

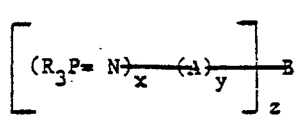

(I)

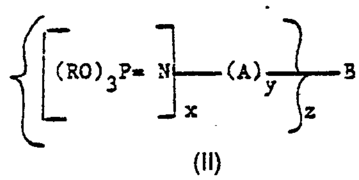

(II)

wobei für z = 1 gilt :

R, B = $C_1$-$C_8$ Alkyl, $C_6$-$C_{10}$ Aryl, $C_7$-$C_{20}$ Alkylaryl oder Arylalkyl,

y = 0 und x = 1, oder

y = 1 und x = 1 für A = —CO—, 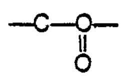 oder —$SO_2$— oder

y = 1 und x = 2 für A = 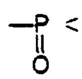

und wobei für z = 2 oder 3 gilt : A, R, x, y entsprechen der o.g. Bedeutung, jedoch B = $C_6$-$C_{10}$ Arylen oder $C_7$-$C_{20}$ Alkylarylen.

Ebenfalls geeignet als erfindungsgemäße Katalysatoren sind Phosphinimine oder Phosphorimidate der allgemeinen Strukturen (III) und (IV)

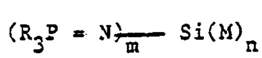

(III)

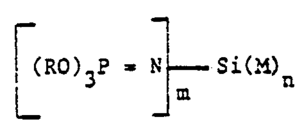

(IV)

wobei R der o.g. Bedeutung entspricht,

M = $CH_3$ oder $C_6H_5$ und

n, m = 1, 2 oder 3, wobei
n + m = 4 sind.
Folgende Einzelverbindungen sind beispielsweise geeignet :

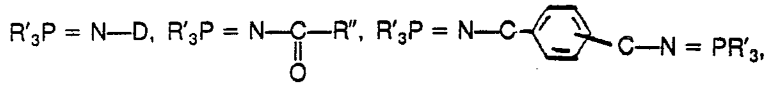

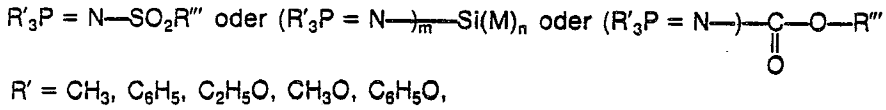

mit R' = $CH_3$, $C_6H_5$, $C_2H_5O$, $CH_3O$, $C_6H_5O$,

D= $CH_3$, $C_6H_5$, 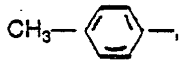, Cyclohexyl,

R'' = $CH_3$, $C_6H_5$, $C_{10}H_7$,

R''' = $C_6H_{13}$, $C_4F_9$, $C_6H_5$, 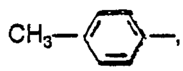, $CH_3$

Die Herstellung der erfindungsgemäß als Katalysatoren geeigneten Phosphinimine der allgemeinen Strukturen (I) und (III) oder Phosphorimidate der allgemeinen Strukturen (II) und (IV) kann beispielsweise durch Umsetzung tertiärer Phosphine oder der Phosphite mit den entsprechenden Aziden unter Stickstoffabspaltung gemäß dem folgenden Reaktionsschema erfolgen (vgl. Kosolapoff, Maier « Organic Phosphorous Compounds », Vol. 3, S. 71-73, s. 127-153, Vol. 6, S. 611-612) :

$$\left[(R_3P = N)_x \text{——} (A)_y\right]_z B \qquad \text{(I)}$$

$$\left.\begin{matrix} z \cdot xR_3P \\ z \cdot x(RO)_3P \end{matrix}\right\} + x \cdot B \left[ (A)_y - N_3 \right]_z \xrightarrow{-z \cdot xN_2} \left\{ \left[ (RO)_3P = N \right]_x (A)_y \right\}_z B \qquad \text{(II)}$$

Besonders geeignets sind folgende Einzelverbindungen :

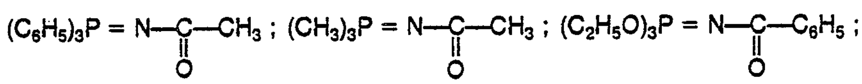

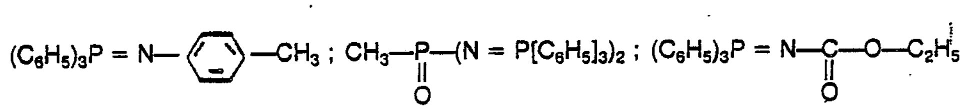

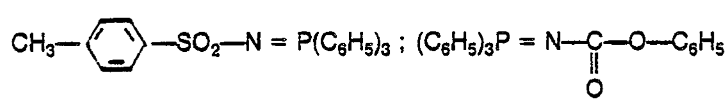

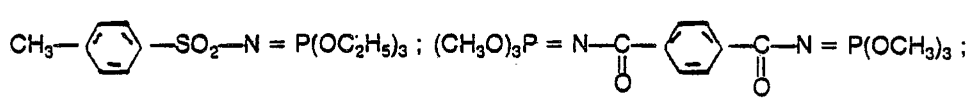

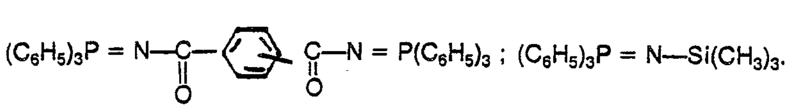

Erfindungsgemäß werden 0,1 bis 3,0 Gew.-%, vorzugsweise 0,2 bis 1,5 Gew.-%, bezogen auf die eingesetzten aromatischen Dicarbonsäuren, an Phosphiniminen oder Phosphorimidaten oder Gemischen an Phosphiniminen oder Phosphorimidaten eingesetzt.

Aromatische Dicarbonsäuren entsprechen den folgenden Formeln :

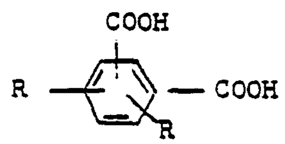

(V)

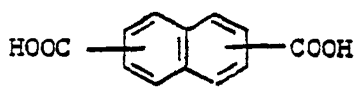

(VI)

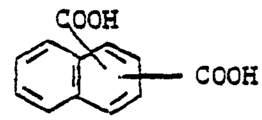

(VII)

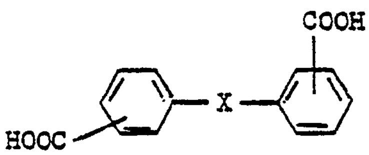

(VIII)

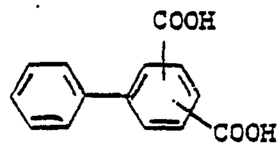

(IX)

mit R = H, $C_1$-$C_4$-Alkyl oder Halogen (bevorzugt Chlor und Brom)
X = einfache Bindung, —O—, —S—.

$$-CH_2, \quad -\underset{CH_3}{\overset{CH_3}{\underset{|}{\overset{|}{C}}}}- , \quad C_5\text{-}C_7 \text{ Cycloalkylen.}$$

Auch Gemische können verwendet werden.

Beispielsweise seien genannt:

Phthalsäure, Isophthalsäure, Terephthalsäure, Gemische aus Iso- und Terephthalsäure, Diphensäure und 1,4-Naphthalindicarbonsäure.

Als Lösungs- oder Verdünnungsmittel werden vorzugsweise das oder die (bei Gemischen) aromatischen Dicarbonsäuredichloride eingesetzt, die während der Reaktion gebildet werden. Inerte Verdünnungsmittel, wie aliphatische oder aromatische Kohlenwasserstoffe, Halogen-substituierte aromatische Kohlenwasserstoffe, Halogen-substituierte aliphatische Kohlenwasserstoffe oder gesättigte aliphatische Ether, können mitverwendet werden. Die Reaktionstemperatur ist im allgemeinen 70 bis 180 °C, vorzugsweise 100 bis 160 °C.

Das Mol-Verhältnis aromatische Dicarbonsäure zu Phosgen ist bevorzugt 1 : 2 bis 1 : 2,5, d.h., ein geringer Überschuß an Phosgen ist ratsam, um Verluste zu ersetzen, die bei der Austreibung von $CO_2$- und HCl-Gas aus dem Reaktionsgemisch während der Phosgenierung entstehen.

Das erfindungsgemäße Verfahren kann diskontinuierlich oder kontinuierlich ausgeführt werden. In einer kontinuierlichen Ausführungsform läßt man in einem Reaktionsrohr eine Lösung aus aromatischer Dicarbonsäure, Dicarbonsäuredichlorid und Katalysator nach unten gegen aufströmendes Phosgengas strömen und nimmt am Boden des Reaktionsrohres aromatisches Dicarbonsäuredichlorid und Katalysator ab.

In einer diskontinuierlichen Ausführungsform werden unter Normaldruck aromatische Dicarbonsäure, aromatisches Dicarbonsäuredichlorid und Katalysator vorgelegt. Dann wird unter Rühren auf 140 bis 160 °C erhitzt, wobei sich die aromatische Dicarbonsäure ganz oder teilweise löst. Bei dieser Temperatur werden pro Mol aromatischer Dicarbonsäure 2 bis 2,5 Mol gasförmiges Phosgen eingeleitet.

Nach Entfernen von überschüssigem Phosgen, HCl- und $CO_2$-Gas durch kurzzeitiges Anlegen von Vakuum erhält man einen Rückstand, der neben der Menge an eingesetztem Katalysator zu ≥ 99,9 % aus aromatischem Dicarbonsäuredichlorid besteht, und der ohne Nachreinigung zu hochmolekularen, farblosen Polykondensaten umgesetzt werden kann.

## Beispiel 1

203 g (1 Mol) Isophthalsäuredichlorid, 166 g Isophthalsäure (1 Mol) und 0,8 g

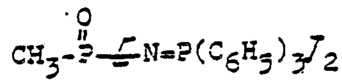

werden im Rundkolben, der mit Thermometer, Rührer und einem durch Kühlsole auf – 20 °C gehaltenen Intensivkühler ausgestattet ist, auf 148 °C erhitzt. Bei 148-157 °C wird unter Rühren und Rückflußsieden so lange Phosgen eingeleitet, bis im Reaktionsgemisch ein Temperaturabfall auf 146 °C eintritt.

Nach Abkühlen auf 120 °C wird Wasserstrahlvakuum angelegt, wodurch überschüssiges Phosgen sowie im Reaktionsgemisch gelöstes HCl- und $CO_2$-Gas entfernt werden.

Ausbeute :

406,5 g eines farblosen Rückstandes, der aus 0,8 g des eingesetzten Phosphinimin-Katalysators und 405,7 g eines 100 %igen Isophthalsäuredichlorids (titrimetrisch bestimmt) besteht.

## Beispiel 2

101,5 g (0,5 Mol) Isophthalsäuredichlorid, 101,5 g (0,5 Mol) Terephthalsäure-dichlorid, 83 g (0,5 Mol) Isophthalsäure, 83 g (0,5 Mol) Terephthalsäure und 0,8 g

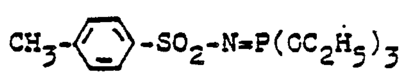

werden, wie im Beispiel 1 beschrieben, mit Phosgen umgesetzt.

Ausbeute :

406,8 g eines farblosen Rückstandes, der zu 0,8 g aus eingesetztem Phosphorimidat-Katalysator und zu 406 g eines Gemisches aus 100 %igem Iso- und Terephthalsäuredichlorid (titrimetrisch bestimmt) besteht.

## Beispiel 3

101,5 g (0,5 Mol) Isophthalsäuredichlorid, 101,5 g (0,5 Mol) Terephthalsäuredichlorid, 83 g (0,5 Mol) Isophthalsäure, 83 g (0,5 Mol) Terephthalsäure und 0,8 g

$$(C_6H_5)_3P = N—CO—CH_3$$

werden, wie im Beispiel 1 beschrieben, mit Phosgen umgesetzt.

Ausbeute :

406 g 99,9 %iges Iso- und Terephthalsäuredichlorid und 0,8 g eingesetzter Phosphinimin-Katalysator.

## Beispiel 4

101,5 g (0,5 Mol) Isophthalsäuredichlorid, 101,5 g (0,5 Mol) Terephthalsäuredichlorid, 83 g (0,5 Mol) Isophthalsäure, 83 g (0,5 Mol) Terephthalsäure und 0,3 g des Katalysators

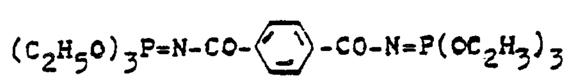

werden, wie im Beispiel 1 beschrieben, mit Phosgen umgesetzt.

Ausbeute :

406 g eines nahezu farblosen Rückstandes aus 0,3 g Katalysator und 405,7 g eines Gemisches aus 100 %igem Iso- und Terephthalsäuredichlorid (titrimetrisch bestimmt).

## Ansprüche

1. Einstufen-Verfahren zur Herstellung von reinen, aromatischen Dicarbonsäuredichloriden durch Umsetzung einer aromatischen Dicarbonsäure oder eines aromatischen Dicarbonsäuregemisches mit Phosgen in Anwesenheit eines Katalysators, und gegebenenfalls in einem Lösungsmittel oder Verdünnungsmittel, dadurch gekennzeichnet, daß man als Katalysatoren Phosphinimine und Phosphorimidate der folgenden allgemeinen Strukturen I bis IV oder deren Mischungen verwendet :

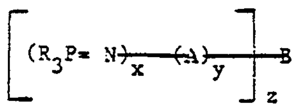

(I)

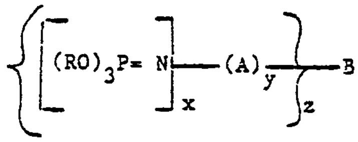

(II)

wobei für z = 1 gilt :

R, B = $C_1$-$C_8$-Alkyl, $C_6$-$C_{10}$-Aryl, $C_7$-$C_{20}$-Alkylaryl oder Arylalkyl,

y = 0 und x = 1, oder

y = 1 und x = 1 für A = —CO—, 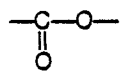 oder —$SO_2$— oder

y = 1 und x = A = 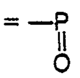

und wobei für z = 2 oder 3 gilt : A, R, x, y entsprechen der o.g. Bedeutung, jedoch B = $C_6$-$C_{10}$-Arylen oder $C_7$-$C_{20}$-Alkylarylen ;

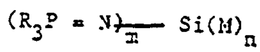

(III)

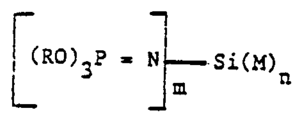

(IV)

wobei

R der o.g. Bedeutung entspricht,

M = $CH_3$ oder $C_6H_5$ und

n, m = 1, 2 oder 3,

wobei

n + m = 4 sind.

2. Verwendung der gemäß Anspruch 1 erhaltenen aromatischen Dicarbonsäuredichloride zur Herstellung von Polykondensaten.

**Claims**

1. A one-stage process for the preparation of pure, aromatic dicarboxylic acid dichlorides by reacting an aromatic dicarboxylic acid or an aromatic dicarboxylic acid mixture with phosgene in the presence of a catalyst, and optionally in a solvent or diluent, characterised in that the catalysts used are phosphine imines and phosphorimidates of the following general formulae I to IV or mixtures thereof :

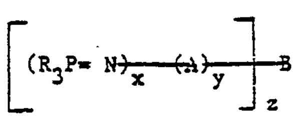

(I)

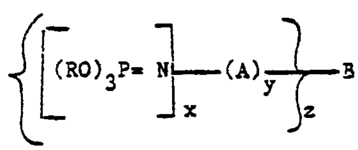

(II)

wherein when z = 1,

R, B = $C_1$-$C_8$-alkyl, $C_6$-$C_{10}$-aryl, $C_7$-$C_{20}$-alkylaryl or arylalkyl,

y = 0 and x = 1, or

y = 1 and x = 1 when A = —CO—, 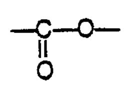 or —$SO_2$— or

y = 1 and x = A = 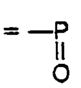

and, when z = 2 or 3, A, R, x, and y have the above meaning, but B = $C_6$-$C_{10}$-arylene or $C_7$-$C_{20}$-alkylarylene ;

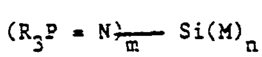

(III)

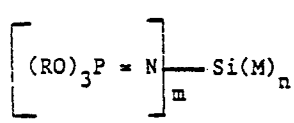

(IV)

wherein

R has the abovementioned meaning,

M = $CH_3$ or $C_6H_5$ and

n, m = 1, 2 or 3,

and

n + m = 4.

2. Use of the aromatic dicarboxylic acid dichlorides obtained according to Claim 1 for the preparation of polycondensates.

**Revendications**

1. Procédé à une étape pour la préparation de dichlorures d'acides dicarboxyliques aromatiques purs par réaction d'un acide dicarboxylique aromatique ou d'un mélange d'acides dicarboxyliques aromatiques avec du phosgène en présence d'un catalyseur et éventuellement dans un solvant ou un diluant, caractérisé en ce que, comme catalyseurs, on utilise des phosphinimines et des phosphorimidates ayant les structures générales I à IV ci-après ou leurs mélanges :

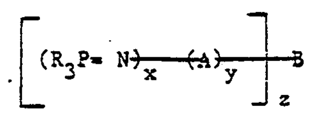

(I)

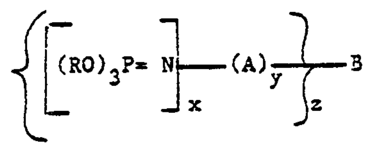

(II)

où lorsque z = 1,

R, B = alkyle en $C_1$-$C_8$, aryle en $C_6$-$C_{10}$, arylalkyle ou alkylaryle en $C_7$-$C_{20}$,

y = 0 et x = 1, ou

y = 1 et x = 1 lorsque A = —CO—, 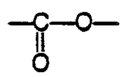 ou —$SO_2$—, ou

y = 1 et x = A = 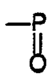

et lorsque z = 2 ou 3, A, R, x et y ont les significations indiquées ci-dessus, mais B = arylène en $C_6$-$C_{10}$ ou alkylarylène en $C_7$-$C_{20}$ ;

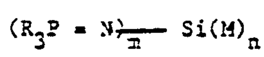

(III)

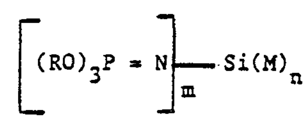

(IV)

où

R a la signification indiquée ci-dessus,

M = $CH_3$ ou $C_6H_5$, et

n, m = 1, 2 ou 3,

n + m = 4.

2. Utilisation des dichlorures d'acides dicarboxyliques aromatiques obtenus suivant la revendication 1 pour la préparation de polycondensats.